(19) Europäisches Patentamt
European Patent Office
Office européen des brevets

(11) Veröffentlichungsnummer: **0 034 115**
**B1**

(12) # EUROPÄISCHE PATENTSCHRIFT

(45) Veröffentlichungstag der Patentschrift: **08.06.83**

(21) Anmeldenummer: **81730012.2**

(22) Anmeldetag: **04.02.81**

(51) Int. Cl.³: **C 07 J 1/00**, C 07 J 5/00, C 07 J 7/00, C 07 J 13/00, C 07 J 21/00, C 07 J 41/00, C 07 J 63/00, C 07 J 71/00

(54) **Verfahren zur Herstellung von 6-Methylensteroiden.**

(30) Priorität: **05.02.80 DE 3004508**

(43) Veröffentlichungstag der Anmeldung: **19.08.81 Patentblatt 81/33**

(45) Bekanntmachung des Hinweises auf die Patenterteilung: **08.06.83 Patentblatt 83/23**

(84) Benannte Vertragsstaaten: **AT BE CH DE FR GB IT LI NL SE**

(56) Entgegenhaltungen:
**FR A 1 563 606**
**JOURNAL OF ORGANIC CHEMISTRY, Band 30, Nr. 9, September 1965, Washington, DC, US W.H.W. LUNN: «The Acid-catalysed reaction between ketones and formaldehyde in dimethyl sulfoxide» Seiten 2925–2930**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Annen, Klaus, Dr., Seegefelderstrasse 194, D-1000 Berlin 20 (DE)**
Erfinder: **Laurent, Henry, Dr., Glambeckerweg 21, D-1000 Berlin 28 (DE)**
Erfinder: **Hofmeister, Helmut, Dr., Weislingenstrasse 4, D-1000 Berlin 28 (DE)**
Erfinder: **Wiechert, Rudolf, Prof.Dr., Petzowerstrasse 8a, D-1000 Berlin 39 (DE)**

EP 0 034 115 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von 6-Methylensteroiden

Die Erfindung betrifft ein Verfahren zur Herstellung von 6-Methylen-$\Delta^4$-3-keto-steroiden der allgemeinen Teilformel I

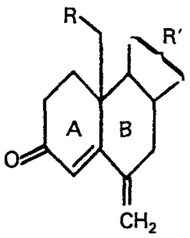

(I), worin

R Wasserstoff, Acyloxy und Alkoxy mit bis zu 6 C-Atomen und

R' das CD-Ringskelett von Steroiden der Androstan- und Pregnanreihe darstellt, das dadurch gekennzeichnet ist, dass man $\Delta^4$-3-Keto-steroide der allgemeinen Teilformel II

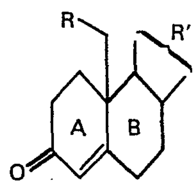

(II), worin

R und R' die oben angegebene Bedeutung haben, mit einem Formaldehydderivat der Formel

$X(CH_2O)_nY$, wobei

X Alkoxy und Y Alkyl mit bis zu 5 C-Atomen bedeuten, wenn n = 1 ist, oder X und Y eine C-O-Einfachbindung bedeuten, wenn n = 3 ist oder X Hydroxy und Y Wasserstoff bedeuten, wenn n eine ganze Zahl in der Grössenordnung von 100 – 1000 ist, in einem inerten Lösungsmittel mit einem Kondensationsmittel bei Temperaturen von 0 – 80 °C behandelt.

Das Teilstück des CD-Ringskeletts von Steroiden kann in an sich bekannter Weise substituiert sein und hat keinen Einfluss auf den Verlauf des erfindungsgemässen Verfahrens. Der D-Ring kann 5-gliedrig (Cyclopentanophenanthrenreihe) oder 6-gliedrig (D-Homo-Reihe) sein. Beispielsweise können die CD-Ringe folgendermassen substituiert sein:

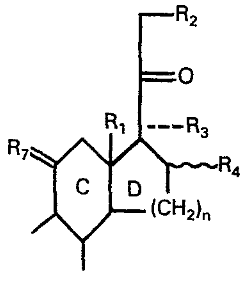

, wobei

n = 1 oder 2 ist,

$R_1$ Methyl oder Ethyl,

$R_2$ Wasserstoff, Acyloxy mit bis zu 12 C-Atomen, Halogen wie Fluor, Chlor oder Brom, Alkoxy mit bis zu 6 C-Atomen oder eine Acetalgruppe der Formel

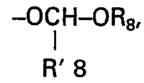

, wobei

$R'_8$ für Wasserstoff oder Alkyl mit bis zu 6 C-Atomen und

$R_8$ für eine Alkylgruppe mit bis zu 6 C-Atomen steht, $R_3$ Wasserstoff, Hydroxy, Alkyl, Alkoxy und Acyloxy mit bis zu 6 C-Atomen, Nitrooxy, und die Acetal- oder Hemithioacetalgruppe der Formel

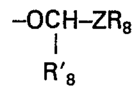

, wobei

$R'_8$ und $R_8$ die oben angegebene Bedeutung haben und Z für Sauerstoff oder Schwefel steht,

$R_4$ Wasserstoff, α-oder β-Alkyl, Alkyliden, α- oder β-Acyloxy, α- oder β-Alkoxy und α- oder β-$OCH_2OR_8$ , wobei $R_8$ die oben angegebene Bedeutung hat und $R_3$ und $R_4$ gemeinsam Sauerstoff, Methylen und die Gruppierung

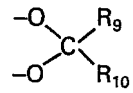

, wobei

$R_9$ und $R_{10}$ gleich oder verschieden sein können und Wasserstoff, Alkyl und Alkoxy mit bis zu 6 C-Atomen bedeuten und

$R_7$ Sauerstoff oder Wasserstoff, Trimethylsilyl, β-Hydroxy, Nitrooxy, β-Halogen wie Fluor oder Chlor, niederes α- oder β-Acyloxy, niederes α- oder β-Alkoxy- α- oder β-Methoxymethoxy und Methylen bedeuten,

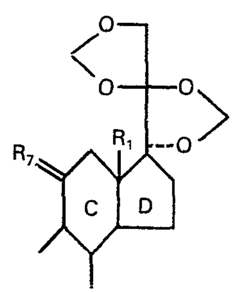

, wobei

$R_1$ und $R_7$ die oben angegebene Bedeutung haben,

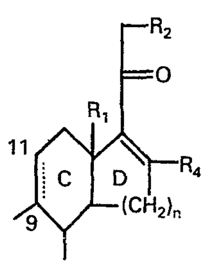

, wobei

$R_1$, $R_2$ und $R_4$ die oben angegebene Bedeutung haben und $C_9 \overset{...}{=} C_{11}$ eine CC-Einfach- oder Doppelbindung bedeutet,

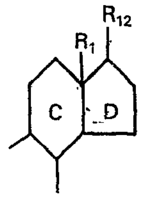

, wobei

$R_1$ die oben angegebene Bedeutung hat und $R_{12}$ für Sauerstoff, Ethylendioxy oder $=C-COOR_{18}$ steht, wobei $R_8$ die oben angegebene Bedeutung hat,

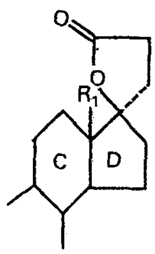

, wobei

$R_1$ die oben angegebene Bedeutung hat,

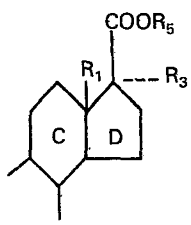

, wobei

$R_1$ und $R_3$ die oben angegebene Bedeutung haben und
$R_5$ Alkyl und Halogenalkyl mit bis zu 6 C-Atomen bedeutet,

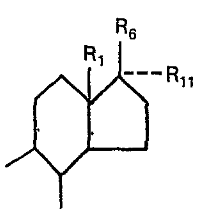

, wobei

$R_1$ die oben angegebene Bedeutung hat und
$R_6$ Hydroxy, Acyloxy mit bis zu 6 C-Atomen, Nitrooxy, Alkoxy mit bis zu 6 C-Atomen und die Acetal- und Hemithioacetalgruppierung der Formel $-\underset{R'_8}{\underset{|}{OCH}}-ZR_8$,
Z die oben angegebene Bedeutung haben und
$R_{11}$ Alkyl, Alkenyl und Alkinyl mit bis zu 6 C-Atomen, das gegebenenfalls mit Fluor, Chlor oder Brom substituiert sein kann, bedeuten und

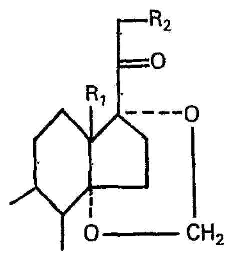

, wobei

$R_1$ und $R_2$ die oben angegebene Bedeutung haben.

Unter Acyloxy mit bis zu 6 bzw. 12 C-Atomen sind solche Säurereste zu verstehen, die sich von Säuren ableiten, die in der Steroidchemie üblicherweise für Veresterungen angewandt werden. Bevorzugte Säuren sind Carbonsäuren mit 1 bis 12 Kohlenstoffatomen. Die Carbonsäuren können auch ungesättigt, verzweigt, mehrbasisch oder in üblicher Weise, zum Beispiel durch Hydroxy-, Amino-, Oxogruppen oder Halogenatome, substituiert sein. Geeignet sind auch cycloaliphatische, aromatische, gemischt aromatisch-aliphatische oder heterocyclische Säuren, die ebenfalls in üblicher Weise substituiert, zum Beispiel durch Halogenatome, sein können. Als bevorzugte Säuren zur Ausbildung des Acylrestes seien beispielsweise genannt: Essigsäure, Propionsäure, Capronsäure, Önanthsäure, Undecylsäure, Ölsäure, Trimethylessigsäure, Trifluoressigsäure, Dichloressigsäure, Cyclopentylpropionsäure, Cyclohexylessigsäure, Phenylpropionsäure, Phenylessigsäure, Phenoxyessigsäure, Dialkylaminessigsäure, Piperidinoessigsäure, Bernsteinsäure, Benzoesäure und andere.

Unter niederem Acyloxy mit bis zu 6 Kohlenstoffatomen sollen Säurereste verstanden sein, die sich von niederen Carbonsäuren ableiten. Genannt seien beispielsweise die Ameisensäure, Essigsäure, Propionsäure, Buttersäure und Capronsäure.

Unter niederem Alkyl bzw. Alkoxy mit bis zu 6 Kohlenstoffatomen sind solche Reste zu verstehen, die sich von den entsprechenden aliphatischen Kohlenwasserstoffen, wie z.B. Methan, Ethan, Propan, i-Propan, n-Butan, i-Butan und tert.-Butan, ableiten.

Die erfindungsgemäss herstellbaren 6-Methylensteroide sind Ausgangsstoffe zur direkten Herstellung von 6-Methylsteroiden. Man erhält diese nach bekannten Methoden aus entsprechenden 3-Keto-$\Delta^4$-6-methylensteroiden durch Isomerisierung, wobei man zu 3-Keto-6-methyl-$\Delta^{4,6}$-steroiden oder durch Hydrierung mit anschliessender Epimerisierung durch Säurebehandlung derselben, wobei man zu 3-Keto-6α-methyl-$\Delta^4$-steroiden gelangt.

Da die Methylgruppe in 6-Stellung oft eine erhebliche Wirkungssteigerung bei Gestagenen und Corticoiden hervorruft, hat es in der Vergangenheit nicht daran gefehlt, nach Wegen zu suchen, die Methylgruppe möglichst einfach in die 6-Stellung einzuführen.

Die bekannten Methoden zur Einführung einer

6-Methylengruppe bei Steroiden sind alle sehr aufwendig und verlaufen über mehrere Stufen.

Die Aufgabe der vorliegenden Erfindung war es, ein Verfahren zu finden, die Methylengruppe in einfacher Weise in die 6-Stellung von verschiedenartigen Steroiden einzuführen, um so ein Ausgangsmaterial zur Herstellung von 6-Methylsteroiden zu erhalten. Die erfindungsgemässe Aufgabe wurde dadurch gelöst, dass man $\Delta^4$-3-Keto-steroide der allgemeinen Teilformel II

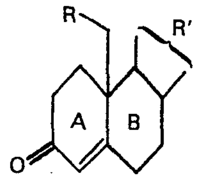

(II), worin

R und R' die oben angegebene Bedeutung haben, mit einem Formaldehydderivat der Formel

$X(CH_2O)_nY$, wobei

X Alkoxy und Y Alkyl mit bis zu 5 C-Atomen bedeuten, wenn n = 1 ist, oder X und Y eine C-O-Einfachbindung bedeuten, wenn n = 3 ist oder X Hydroxy und Y Wasserstoff bedeuten, wenn n eine ganze Zahl in der Grössenordnung von 100–1000 ist, in einem inerten Lösungsmittel mit einem Kondensationsmittel bei Temperaturen von 0–80 °C behandelt.

Die erfindungsgemässe Reaktion wird in einem inerten Lösungsmittel ausgeführt. Beispielsweise genannt sei Methylenchlorid, Chloroform, Tetrachlorkohlenstoff, 1.2-Dichloräthan, Diethylether, Dioxan, Tetrahydrofuran und Petroläther.

Die Lösungsmittel können auch als Gemische untereinander verwendet werden.

Als Kondensationsmittel sind an sich alle Phosphorsäurederivate wie Phosphorpentoxid, Phosphoroxychlorid, Phosphoroxytribromid, Ethyldichlorphosphat und Phosphorpentachlorid geeignet. Geeignet sind aber auch starke Säuren wie konzentrierte Schwefelsäure, Salzsäure, p-Toluolsulfonsäure und Perchlorsäure sowie stark saure Kationenaustauscher auf Kunstharzbasis wie Polystyrol, Epoxiharz oder Polyacrylat. Als bevorzugte Mittel haben sich phosphorpentoxid und Phosphoroxychlorid bewährt.

Obwohl sich die erfindungsgemässe Reaktion in einfacher Weise mit den genannten Kondensationsmitteln ausführen lässt, empfiehlt es sich bei der Verwendung von Phosphorpentoxid ein Trägermaterial einzusetzen, um eine bessere Verteilung des Kondensationsmittels zu erreichen. Geeignete Trägermaterialien sind beispielsweise Kieselgur, Kieselgel, Celite, Aluminiumoxid, Bleicherde, Bentonite und Graphit. Bei der Verwendung von Halogenphosphorsäurederivaten ist der Zusatz eines Puffers zweckmässig. Geeignete Puffersubstanzen sind Alkalisalze schwacher organischer Säuren wie z.B. Natriumacetat, Kaliumacetat und Natriumcitrat und Salze der Phosphorsäure wie z.B. Kaliumdihydrogenphosphat und Dinatriumhydrogenphosphat.

Die Menge des zugesetzten Trägermaterials und/oder Puffersubstanz ist unkritisch. Das Kondensationsmittel wird in einer Menge von 0,5–10, vorzugsweise 1–2 Moläquivalenten eingesetzt.

Der Verlauf der erfindungsgemässen Reaktion war insofern überraschend, da eine direkte Kondensation von $\Delta^4$-3-Ketosteroiden mit Formaldehyd bzw. den verwendeten Formaldehydderivaten zu den entsprechenden 6-Methylen-$\Delta^4$-3-ketosteroiden für den Fachmann nicht vorhersehbar war.

Das erfindungsgemässe Verfahren hat den Vorteil, dass es über weniger Stufen verläuft als bekannte Verfahren zur Einführung einer Methylen- bzw. Methylgruppe in 6-Stellung von 3-Keto-$\Delta^4$-steroiden. Eine 3.5-Dienoletherbildung, wie sie z.B. von Petrow et al., Tetrahedron, 21 (1965)1624, oder eine 3.5-Dienaminbildung des 3-Keto-$\Delta^4$-Systems vor der elektrophilen CC-Verknüpfung am Kohlenstoffatom C-6, wie sie z.B. bei Fürst et al., Helv.Chim. Acta 56 (1973)2396 beschrieben wird, ist nicht notwendig. Oder ein Schutz der 3-Ketogruppe vor einer 5α.6α-Epoxidringöffnung mit Grignard-Reagentien, wie sie z.B. von Spero et al., J.Amer.Chem.Soc. 78 (1956)6213 beschrieben wird, entfällt.

Der Vorteil des erfindungsgemässen Verfahrens liegt somit darin, dass man direkt in einem Schritt von einem $\Delta^4$-3-Ketosteroid zu einem $\Delta^4$-3-Keto-6-methylensteroid gelangt, das gewünschtenfalls dann in an sich bekannter Weise zu den bekannten $\Delta^4$-3-Keto-6α-methylsteroiden hydriert bzw. zu den bekannten $\Delta^{4,6}$-3-Keto-6-methylsteroiden isomerisiert werden kann.

Die nachfolgenden Beispiele sollen das erfindungsgemässe Verfahren erläutern.

Beispiel 1

Eine Lösung von 5,0 g 17.21-Diacetoxy-4-pregnen-3.20-dion in 35 ml wasserfreiem Methylenchlorid und 22,5 ml Methylal wird bei Raumtemperatur mit einem Gemisch aus 1,0 g Phosphorpentoxid und 7,5 g Kieselgel portionsweise versetzt. Man rührt 3 Tage bei Raumtemperatur und fügt weitere 11 ml Methylal, 500 mg Phosphorpentoxid und 3,75 g Kieselgel hinzu. Nach weiteren 2 Tagen saugt man das Reaktionsgemisch ab, wäscht den Rückstand gründlich mit Methylenchlorid und engt im Vakuum zur Trockne ein. Das Rohprodukt wird an 350 g Kieselgel mit einem Hexan-Essigester Gradienten (0–50% Essigester) gereinigt. Man erhält 3,6 g 17α.21-Diacetoxy-6-methylen-4-pregnen-3.20-dionvom Schmelzpunkt

227–228 °C. $[\alpha]_D^{25} = +165°$ (Chloroform)

Beispiel 2

5,0 g 17α-Acetoxy-4-pregnen-3.20-dion werden analog Beispiel 1 zu 2,7 g 17α-Acetoxy-6-methylen-4-pregnen-3.20-dion umgesetzt.

Schmelzpunkt 232,5–234 °C.

Beispiel 3

2,0 g 17β-Acetoxy-4-androsten-3-on werden unter den Bedingungen des Beispiel 1 zu 860 mg

17β-Acetoxy-6-methylen-4-androsten-3-on umgesetzt und gereinigt.

Schmelzpunkt 138–140 °C.

Beispiel 4

5,0 g 21-Acetoxy-17α-nitrooxy-4-pregnen-3.20-dion werden analog Beispiel 1 zu 2,4 g 21-Acetoxy-6-methylen-17α-nitrooxy-4-pregnen-3.20-dion umgesetzt.

Schmelzpunkt 136–137 °C.

Beispiel 5

Unter den Bedingungen des Beispiels 1 werden 5,0 g 21-Acetoxy-11β.17α-dinitrooxy-4-pregnen-3.20-dion zu 2,6 g 21-Acetoxy-6-methylen-11β.17α-dimitrooxy-4-pregnen-3.20-dion umgesetzt.

Schmelzpunkt 167,5–171 °C (Zersetzung).

Beispiel 6

Analog Beispiel 1 werden 5,0 g 21-Acetoxy-4.16-pregnadien-3.20-dion zu 2,3 g 21-Acetoxy-6-methylen-4.16-pregnadien-3.20-dion umgesetzt.

Schmelzpunkt 159,5–161 °C.

Beispiel 7

Es werden 5,0 g 4-Androsten-[17(β-1')-spiro-5']-perhydrofuran-2'.3-dion unter den Bedingungen des Beispiels 1 zu 2,5 g 6-Methylen-4-androsten-[17(β-1')-spiro-5']-perhydrofuran-2'.3-dion umgesetzt und gereinigt.

Schmelzpunkt 142–143 °C.

Beispiel 8

Ein Gemisch von 17α.21-Diacetoxy-4-pregnen-3.20-dion und 3.17α.21-Triacetoxy-3.5-pregnadien-20-on, das aus 5,0 g 21-Acetoxy-17α-hydroxy-4-pregnen-3.20-dion in 63 ml Diethylenglykoldimethylether mit 7,4 g N.N-Dimethylaminopyridin und 7,4 ml Essigsäureanhydrid durch 6,5 stündiges bei 80 °C Rühren hergestellt wurde, wird unter den Bedingungen des Beispiels 1 zu 2,4 g 17α.21-Diacetoxy-6-methylen-4-pregnen-3.20-dion umgesetzt.

Schmelzpunkt 227–228 °C.

Beispiel 9

Zu einer Suspension von 1,0 g Natriumacetat in 30 ml Methylenchlorid, 30 ml Formaldehyddimethylacetal und 3,8 ml Phosphoroxychlorid gibt man 1,0 g 17.21-Diacetoxy-4-pregnen-3.20-dion und refluxiert 35 Stunden unter Rühren. Nach dem Abkühlen auf Raumtemperatur verdünnt man mit Methylenchlorid und Wasser. Man tropft unter Rühren eine gesättigte Sodalösung bis zur alkalischen Reaktion der wässrigen Phase hinzu. Die organische Phase wird abgetrennt, mit Wasser gewaschen, über Natriumsulfat getrocknet und im Vakuum eingeengt. Schliesslich kristallisiert man aus Aceton/Hexan um und isoliert 825 mg 17.21-Diacetoxy-6-methylen-4-pregnen-3.20-dion vom Schmelzpunkt 227–228 °C.

Beispiel 10

Analog Beispiel 1 wird 1,0 g 19-Acetoxy-17-hexanoyloxy-4-pregnen-3.20-dion in 7 ml Methylenchlorid mit 4,9 ml Formaldehyddimethylacetal und einem Gemisch aus 200 mg Phosphorpentoxid und 1,5 g Kieselgur W 20 umgesetzt und aufgearbeitet. Das Rohprodukt wird an 110 g Kieselgel mit einem Hexan/Aceton-Gradienten (0–20% Aceton) gereinigt. Man isoliert 563 mg 19-Acetoxy-17-hexanoyloxy-6-methylen-4-pregnen-3.20-dion vom Schmelzpunkt 111–112 °C.

Beispiel 11

Analog Beispiel 9 werden 2,0 g 17.21-Diacetoxy-4-pregnen-3.20-dion in 60 ml Methylenchlorid mit 60 ml Formaldehyddimethylacetal, 2,0 g Natriumacetat und 12,6 g Phosphoroxybromid umgesetzt und aufgearbeitet. Man isoliert 1,68 g 17.21-Diacetoxy-6-methylen-4-pregnen-3.20-dion vom Schmelzpunkt 226–228 °C.

Beispiel 12

Eine Lösung von 2,0 g 17.21-Diacetoxy-4-pregnen-3.20-dion wird in 60 ml Methylenchlorid und 60 ml Formaldehyddimethylacetal mit 2,0 g Natriumacetat und 12,6 g Phosphorpentachlorid 30 Stunden refluxiert. Man arbeitet analog Beispiel 9 auf und isoliert 1,21 g 17.21-Diacetoxy-6-methylen-4-pregnen-3.20-dion vom Schmelzpunkt 227–228 °C.

Beispiel 13

Analog Beispiel 9 wird eine Lösung von 2,0 g 17.21-Diacetoxy-4-pregnen-3.20-dion und 5,0 g Paraformaldehyd in 60 ml Methylenchlorid und 2,0 g Natriumacetat und 7,6 ml Phosphoroxychlorid umgesetzt und aufgearbeitet. Man isoliert 1,05 g 17.21-Diacetoxy-6-methylen-4-pregnen-3.20-dion vom Schmelzpunkt 225,5–228 °C.

Beispiel 14

1,0 g 17.21-Diacetoxy-4-pregnen-3.20-dion werden analog Beispiel 9 mit Formaldehyddiethylacetal umgesetzt und aufgearbeitet. Man erhält 790 mg 17.21-Diacetoxy-6-methylen-4-pregnen-3.20-dion vom Schmelzpunkt 227–228 °C.

Beispiel 15

Unter den Bedingungen des Beispiels 9 wird eine Lösung von 1,0 g 17.21-Diacetoxy-4-pregnen-3.20-dion und 2,5 g Trioxan in 30 ml Methylenchlorid mit 1,0 g Natriumacetat und 3,8 ml Phosphoroxychlorid umgesetzt und aufgearbeitet. Man erhält 530 mg 17.21-Diacetoxy-6-methylen-4-pregnen-3.20-dion vom Schmelzpunkt 225,5–228 °C.

Beispiel 16

2,0 g 4-Androsten-3.17-dion werden unter den Bedingungen des Beispiels 9 zu 1,41 g 6-Methylen-4-androsten-3.17-dion umgesetzt.

Schmelzpunkt 163–164 °C.

Beispiel 17

Es werden 2,0 g 4-Pregnen-3.20-dion unter den

Bedingungen des Beispiels 9 zu 1,84 g 6-Methylen-4-pregnen-3.20-dion umgesetzt.

Schmelzpunkt 131–132 °C.

Beispiel 18

Analog Beispiel 9 werden 2,0 g 21-Acetoxy-4-pregnen-3.20-dion zu 1,72 g 21-Acetoxy-6-methylen-4-pregnen-3.20-dion umgesetzt.

Schmelzpunkt 112,5–114 °C.

Beispiel 19

1,0 g 17α-Acetoxy-21-äthoxyacetoxy-D-homo-4-pregnen-3.20-dion werden analog Beispiel 1 zu 680 mg 17α-Acetoxy-21-äthoxyacetoxy-6-methylen-D-homo-4-pregnen-3.20-dion umgesetzt.

Schmelzpunkt 141,5–143 °C.

Beispiel 20

Unter den Bedingungen des Beispiels 9 werden 2,0 g 17β-Acetoxy-17α-methyl-4-androsten-3-on zu 1,58 g 17β-Acetoxy-17α-methyl-6-methylen-4-androsten-3-on umgesetzt.

Schmelzpunkt 142–144 °C.

Beispiel 21

2,0 g 17β-Propionyloxy-17α-vinyl-4-androsten-3-on werden analog Beispiel 9 zu 1,32 g 6-Methylen-17β-propionyloxy-17α-vinyl-4-androsten-3-on umgesetzt.

Schmelzpunkt 127–130 °C.

Beispiel 22

Analog Beispiel 9 wird 1,0 g 17α-(1-propinyl)-17β-propionyloxy-4-androsten-3-on zu 635 mg 6-Methylen-17α-(1-propinyl)-17β-propionyloxy-4-androsten-3-on umgesetzt.

Schmelzpunkt 139–140 °C.

Beispiel 23

Es werden 3,0 g 17α-Acetoxy-21-fluor-4-pregnen-3.20-dion unter den Bedingungen des Beispiels 9 zu 2,36 g 17α-Acetoxy-21-fluor-6-methylen-4-pregnen-3.20-dion umgesetzt.

Schmelzpunkt 202,5–205 °C.

Beispiel 24

Unter den Bedingungen des Beispiels 9 werden 1,5 g 17α-Acetoxy-16-methylen-4-pregnen-3.20-dion zu 910 mg 17α-Acetoxy-6.16-dimethylen-4-pregnen-3.20-dion umgesetzt.

Schmelzpunkt 220–222 °C.

Beispiel 25

2,0 g 16α.17α-Methylen-4-pregnen-3.20-dion werden analog Beispiel 9 zu 1,59 g 6-Methylen-16α.17α-methylen-4-pregnen-3.20-dion umgesetzt.

Schmelzpunkt 166,5–168 °C.

Beispiel 26

Analog Beispiel 9 werden 2,0 g 16α.17α-Isopropylidendioxy-4-pregnen-3.20-dion zu 1,73 g 16α.17α-Isopropylidendioxy-6-methylen-4-pregnen-3.20-dion umgesetzt.

Schmelzpunkt 223–224,5 °C.

Beispiel 27

Es werden 1,5 g 16α.17α-Epoxy-4-pregnen-3.20-dion unter den Bedingungen des Beispiels 9 zu 863 mg 16α.17α-Epoxy-6-methylen-4-pregnen-3.20-dion umgesetzt.

Schmelzpunkt 185–187 °C.

Beispiel 28

Unter den Bedingungen des Beispiels 9 werden 2,0 g 3-Oxo-4.17(20)-pregnadien-21-säure-ethylester zu 1,26 g 6-Methylen-3-oxo-4.17(20)-pregnadien-21-säure-ethylester umgesetzt.

Schmelzpunkt 161,5–162 °C.

Beispiel 29

2,0 g 17α.21-Diacetoxy-4-pregnen-3.11.20-trion werden analog Beispiel 9 zu 1,76 g 17α.21-Diacetoxy-6-methylen-4-pregnen-3.11.20-trion umgesetzt.

Schmelzpunkt 228–230 °C.

Beispiel 30

Analog Beispiel 9 werden 2,0 g 17α.20;20.21-Bismethylendioxy-4-pregnen-3.11-dion zu 1,54 g 6-Methylen-17α.20;20.21-bismethylendioxy-4-pregnen-3.11-dion umgesetzt.

Schmelzpunkt 198,5–201 °C.

Beispiel 31

Es wird 1,0 g 17α.21-Diacetoxy-4.9(11)-pregnadien-3.20-dion analog Beispiel 1 zu 723 mg 17α.21-Diacetoxy-6-methylen-4.9(11)-pregnadien-3.20-dion umgesetzt.

Schmelzpunkt 208,5–211 °C.

Beispiel 32

Unter den Bedingungen des Beispiels 9 werden 2,0 g 21-Acetoxy-4.9(11).16-pregnatrien-3.20-dion zu 1,15 g 21-Acetoxy-6-methylen-4.9(11).16-pregnatrien-3.20-dion umgesetzt.

Schmelzpunkt 155–157 °C.

Beispiel 33

1,0 g 21-Acetoxy-17α-methoxy-4-pregnen-3.20-dion wird analog Beispiel 9 umgesetzt. Man erhält 0,58 g 21-Acetoxy-17α-methoxy-6-methylen-4-pregnen-3.20-dion vom Schmelzpunkt 141,5–143 °C.

Beispiel 34

0,5 g 17α-Hexanoyloxy-19-methoxy-4-pregnen-3.20-dion werden analog Beispiel 9 umgesetzt und 0,31 g 17α-Hexanoyloxy-19- methoxy-6-methylen-4-pregnen-3.20-dion vom Schmelzpunkt 94–96 °C werden erhalten.

Beispiel 35

In einem 2-Halskolben wird eine Suspension von 1,0 g Natriumacetat in 30 ml Chloroform, 30 ml Formaldehyddiethylacetal und 3,8 ml Phosphoroxychlorid 1 Stunde bei 65 °C Badtemperatur gerührt und anschliessend mit 1,0 g 17α-Hydroxyprogesteron versetzt. Man rührt 1,5 Stunden bei 65 °C weiter, kühlt auf Raumtemperatur ab und tropft unter kräftigem Rühren eine gesättigte Sodalösung bis zur alkalischen Reaktion hinzu. Die organische Phase wird abgetrennt, mit Wasser neutral gewaschen, über Natriumsulfat getrocknet und im Vakuum eingeengt. Das Rohprodukt wird an 220 g Kieselgel mit einem Methylenchlorid/Aceton-Gradienten (0–12% Aceton) gereinigt. Man erhält 842 mg 17α-Hydroxy-6-methylen-4-pregnen-3.20-dion vom Schmelzpunkt 218–220 °C.

Beispiel 36

Analog Beispiel 35 wird 1,0 g 21-Acetoxy-17α-hydroxy-4-pregnen-3.20-dion mit einer Suspension von 1,0 g Natriumacetat in 30 ml Chloroform, 30 ml Formaldehyddiethylacetal und 3,8 ml Phosphoroxychlorid 7 Stunden bei 65 °C gerührt und anschliessend aufgearbeitet. Das Rohprodukt wird an 220 g Kieselgel mit einem Methylenchlorid/Aceton-Gradienten (0–12% Aceton) gereinigt. Man erhält 890 mg 21-Acetoxy-17α-hydroxy-6-methylen-4-pregnen-3.20-dion vom Schmelzpunkt 194,5–196 °C.

Beispiel 37

1,0 g 21-Acetoxy-17α-hydroxy-D-homo-4-pregnen-3.20-dion wird analog Beispiel 35 zu 790 mg 21-Acetoxy-17α-hydroxy-6-methylen-D-homo-4-pregnen-3.20-dion umgesetzt.

Schmelzpunkt 187–188 °C.

Beispiel 38

In einem 2-Halskolben wird eine Suspension von 1,0 g Natriumacetat, 1,0 g 21-Acetoxy-17α-hydroxy-4.9-pregnadien-3.20-dion in 30 ml Chloroform, 30 ml Formaldehyddiethylacetal und 3,8 ml Phosphoroxychlorid 1 Stunde bei 65 °C Badtemperatur gerührt. Man kühlt auf Raumtemperatur ab und tropft unter kräftigem Rühren eine gesättigte Sodalösung bis zur alkalischen Reaktion hinzu. Die organische Phase wird abgetrennt, mit Wasser neutral gewaschen, über Natriumsulfat getrocknet und im Vakuum eingeengt. Das Rohprodukt wird an 200 g Kieselgel mit einem Hexan-Essigester-Gradienten (0–50% Essigester) gereinigt. Es werden 742 mg 21-Acetoxy-17α-hydroxy-6-methylen-4.9-pregnadien-3.20-dion vom Schmelzpunkt 191–193 °C erhalten.

Beispiel 39

Analog Beispiel 38 wird 1,0 g 17β-Hydroxy-17α-methyl-4-androsten-3-on mit einer Suspension von 1,0 g Natriumacetat in 30 ml Chloroform, 30 ml Formaldehyddiethylacetal und 3,8 ml Phosphoroxychlorid umgesetzt und aufgearbeitet. Es werden 590 mg 17β-Hydroxy-17α-methyl-6-methylen-4-androsten-3-on vom Schmelzpunkt 147,5–150 °C erhalten.

Beispiel 40

Unter den Bedingungen des Beispiel 38 werden 2,0 g 17α-Ethinyl-17β-hydroxy-4-androsten-3-on zu 1,45 g 17α-Ethinyl-17β-hydroxy-6-methylen-4-androsten-3-on umgesetzt und aufgearbeitet.

Schmelzpunkt 243–245 °C.

Beispiel 41

Eine Suspension von 1,0 g Natriumacetat in 30 ml Chloroform und 30 ml Methylal wird mit 1,9 ml Phosphoroxychlorid versetzt und 1 Stunde refluxiert. Man fügt 1,0 g 17β-Acetoxy-17α-ethinyl-4-androsten-3-on hinzu und tropft unter Rückfluss innerhalb von 2 Stunden 1,9 ml Phosphoroxychlorid hinzu. Das Gemisch wird 1 Stunde refluxiert, abgekühlt und durch Zutropfen von gesättigter Sodalösung alkalisch gemacht. Die organische Phase wird abgetrennt und im Vakuum eingeengt. Zur Aufreinigung versetzt man das Rohprodukt mit wenig Methylenchlorid und Wasser sowie 5 g Soda. Nach Wasserdampfdestillation saugt man ab, wäscht den Rückstand mit Wasser neutral und reinigt ihn an 105 g Kieselgel mit einem Hexan-Essigester-Gradienten (0–50% Essigester). Man isoliert 630 mg 17β-Acetoxy-17α-ethinyl-6-methylen-4-androsten-3-on vom Schmelzpunkt 158–160 °C.

Beispiel 42

Zu einer Suspension von 1,0 g Natriumacetat in 30 ml Chloroform, 30 ml Formaldehyddiethylacetal und 3,8 ml Phosphoroxychlorid gibt man 1,0 g 5-Androsten-7.17-dion und refluxiert 6 1/2 Stunden. Man tropft unter Rühren eine gesättigte Sodalösung bis zur alkalischen Reaktion der wässrigen Phase hinzu. Die organische Phase wird abgetrennt, mit Wasser gewaschen, über Natriumsulfat getrocknet und im Vakuum eingeengt. Das Rohprodukt wird an 200 g Kieselgel mit einem Hexan-Essigester-Gradienten (0–50% Essigester) gereinigt. Man isoliert 840 mg 4-Methylen-5-androsten-7.17-dion vom Schmelzpunkt 131,5–132 °C.

Beispiel 43

Analog Beispiel 42 wird 1,0 g 21-Acetoxy-17aα-hydroxy-D-homo-4-pregnen-3.20-dion zu 640 mg 21-Acetoxy-17aα-hydroxy-6-methylen-D-homo-4-pregnen-3.20-dion umgesetzt, aufgearbeitet und gereinigt.

Schmelzpunkt 187–189 °C.

Beispiel 44

1,0 g D-Homo-4.17-pregnadien-3.20-dion wird unter den Bedingungen des Beispiels 42 umgesetzt und aufgereinigt. Man isoliert 530 mg 6-Methylen-D-homo-4.17-pregnadien-3.20-dion vom Schmelzpunkt 138–139 °C.

Beispiel 45

Unter den Bedingungen des Beispiels 42 werden 2,0 g 21-Acetoxy-9α-fluor-17-hydroxy-11β-trifluoracetoxy-4-pregnen-3.20-dion zu 1,1 g 21-Acetoxy-9α-fluor-17-hydroxy-6-methylen-11β-trifluoracetoxy-4-pregnen-3.20-dion umgesetzt und aufbereitet.

Schmelzpunkt 116,5–117 °C.

Beispiel 46

0,5 g 21-Acetoxy-14α.17α-dihydroxy-4-pregnen-3.20-dion werden analog Beispiel 1 zu 0,18 g 21-Acetoxy-6-methylen-14α.17-methylendioxy-4-pregnen-3.20-dion umgesetzt und aufgereinigt.
Schmelzpunkt 171–174 °C.

Beispiel 47

Analog Beispiel 42 werden 3,0 g 21-Acetoxy-17α-hydroxy-11β-trimethylsilyloxy-4-pregnen-3.20-dion zu 670 mg 21-Acetoxy-11β.17α-dihydroxy-6-methylen-4-pregnen-3.20-dion umgesetzt und aufgereinigt.
Schmelzpunkt 203–205 °C.

Beispiel 48

Es wird analog Beispiel 42 1,0 g 21-Chlor-16α.17-isopropylidendioxy-4.9(11)-prognadien-3.20-dion zu 230 mg 21-Chlor-16α.17-isopropylidendioxy-6-methylen-4.9(11)-pregnadien-3.20-dion umgesetzt und aufgereinigt.
Schmelzpunkt 239–242 °C.

Beispiel 49

Analog Beispiel 42 wird 1,0 g 17α-Acetoxy-4-pregnen-3.20-dion mit 3,8 ml Ethyldichlorphosphat zu 510 mg 17α-Acetoxy-6-methylen-4-pregnen-3.20-dion umgesetzt und aufbereitet.
Schmelzpunkt 231–233 °C.

Beispiel 50

1,0 g 21-Acetoxy-17α-hydroxy-4-pregnen-3.20-dion wird unter Verwendung von 2,0 g p-Toluolsulfonsäure analog Beispiel 42 zu 680 mg 21-Acetoxy-17α-hydroxy-6-methylen-4-pregnen-3.20-dion umgesetzt und aufgereinigt.
Ausbeute 680 mg; Schmelzpunkt 194–196 °C.

Beispiel 51

Analog Beispiel 42 wird unter Verwendung von 0,2 ml konzentrierter Schwefelsäure aus 1,0 g 17α-Acetoxy-4-pregnen-3.20-dion 490 mg 17α-Acetoxy-6-methylen-4-pregnen-3.20-dion vom Schmelzpunkt 234–236 °C erhalten.

Beispiel 52

1,0 g 17-Acetoxy-4-pregnen-3.20-dion werden analog Beispiel 42 mit 20 ml konzentrierter Salzsäure zu 410 mg 17-Acetoxy-6-methylen-4-pregnen-3.20-dion umgesetzt und aufbereitet.
Schmelzpunkt 230–234 °C.

Beispiel 53

Unter den Bedingungen des Beispiels 42 wird unter Verwendung von 15 g eines stark sauren Kationenaustauscher auf Polyacrylat-Basis 1,0 g 17-Acetoxy-4-pregnen-3.20-dion zu 320 mg 17-Acetoxy-6-methylen-4-pregnen-3.20-dion umgesetzt und aufbereitet.
Schmelzpunkt 236–238 °C.

Beispiel 54

Unter den Bedingungen des Beispiels 42 wird mit 35 ml Formaldehyddiisopropylacetal 1,0 g 17α-Acetoxy-4-pregnen-3.20-dion zu 480 mg 17α-Acetoxy-6-methylen-4-pregnen-3.20-dion umgesetzt und aufgearbeitet.
Schmelzpunkt 233,5–235 °C.

Beispiel 55

Aus 1,0 g 17α-Acetoxy-4-pregnen-3.20-dion werden analog Beispiel 42 unter Verwendung von 0,2 ml 70%iger Perchlorsäure 450 mg 17α-Acetoxy-6-methylen-4-pregnen-3.20-dion vom Schmelzpunkt 234–236,5 °C erhalten.

**Patentansprüche**

1. Verfahren zur Herstellung von 6-Methylen-$\Delta^4$-3-keto-steroiden der allgemeinen Teilformel I

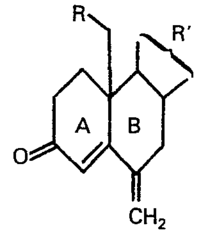

(I), worin

R Wasserstoff, Acyloxy und Alkoxy mit bis zu 6 C-Atomen und

R' das CD-Ringskelett von Steroiden der Androstan- und Pregnanreihe darstellt, dadurch gekennzeichnet, dass man $\Delta^4$-3-Keto-steroide der allgemeinen Teilformel II

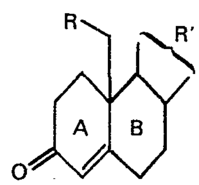

(II), worin

R und R' die oben angegebene Bedeutung haben, mit einem Formaldehydderivat der Formel

$$X(CH_2O)_nY, \text{ wobei}$$

X Alkoxy und Y Alkyl mit bis zu 5 C-Atomen bedeuten, wenn n = 1 ist, oder X und Y eine C–O-Einfachbindung bedeuten, wenn n = 3 ist oder X Hydroxy und Y Wasserstoff bedeuten, wenn n eine ganze Zahl in der Grössenordnung von 100–1000 ist, in einem inerten Lösungsmittel mit einem Kondensationsmittel bei Temperaturen von 0–80 °C behandelt.

2. Verfahren nach Anspruch 1, dadurch gekennzeichnet, dass man als Kondensationsmittel Phosphorpentoxid auf einem Trägermaterial verwendet.

3. Verfahren nach Anspruch 1, dadurch gekennzeichnet, dass man als Kondensationsmittel Phosphoroxychlorid mit einem Zusatz an Natriumacetat verwendet.

**Revendications**

1. Procédé de préparation de 6-méthylène-$\Delta^4$-3-céto-stérostéroïdes répondant à la formule partielle générale I

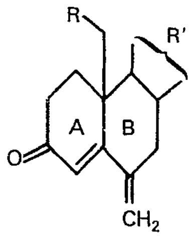

(I),

dans laquelle R représente un atome d'hydrogène ou un radical acyloxy ou alcoxy comprenant jusqu'à 6 atomes de carbone, et R' représente le squelette des noyaux CD de stéroïdes de la série de l'androstane et de la série du prëgnane, caractérisé en ce que, à des températures de 0 à 80 °C, on traite des $\Delta^4$-3-céto-stéroïdes répondant à la formule partielle générale II

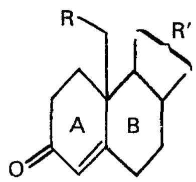

(II),

dans laquelle R et R' ont la même signification que celle donnée ci-dessus, avec un dérivé de formaldéhyde de la formule

$$X(CH_2O)_nY,$$

dans laquelle X représente un radical alcoxy et Y un radical alkyle comprenant jusqu'à 5 atomes de carbone, lorsque n est égal à 1, X et Y représentant une simple liaison carbone-oxygène, lorsque n est égal à 3, et X représentant un groupe hydroxy et Y un atome d'hydrogène, lorsque n est un nombre entier de l'ordre de grandeur de 100 à 1000, dans un solvant inerte, avec un agent de condensation.

2. Procédé suivant la revendication 1, caractérisé en ce qu'on utilise, comme agent de condensation, du pentoxyde de phosphore sur un support.

3. Procédé suivant la revendication 1, caractérisé en ce qu'on utilise, comme agent de condensation, de l'oxychlorure de phosphore avec une addition d'acétate de sodium.

**Claims**

1. Process for the manufacture of 6-methylene-$\Delta^4$-3-ketosteroids of the general partial formula I

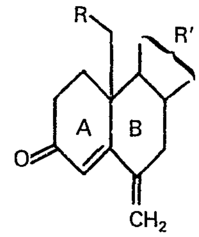

(I)

in which

R represents hydrogen, acyloxy and alkoxy having up to 6 carbon atoms, and

R' represents the CD ring skeleton of steroids of the androstane and pregnane series, characterised in that $\Delta^4$-3-ketosteroids of the general partial formula II

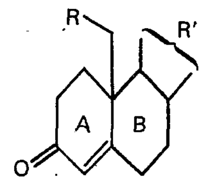

(II)

in which

R and R' have the meanings given above are treated at temperatures of from 0 to 80 °C in an inert solvent containing a condensing agent with a formaldehyde derivative of the formula

$$X(CH_2O)_nY$$

in which, if n is 1, X represents alkoxy and Y represents alkyl having up to 5 carbon atoms, or if n is 3, X and Y represent a C-O single bond, or, if n is an integer of the order of magnitude of from 100 to 1000, X represents hydroxy and Y represents hydrogen.

2. Process according to claim 1, characterised in that phosphorus pentoxide on a carrier material is used as condensing agent.

3. Process according to claim 1, characterised in that phosphorus oxychloride having an additive of sodium acetate is used as condensing agent.